# FASCICULE DE BREVET EUROPEEN

(11) **EP 0 379 409 B1**
(45) Date de publication et mention de la délivrance du brevet: **15.09.1993**
(21) Numéro de dépôt: 90400099.9
(22) Date de dépôt: 15.01.1990
(51) Int. Cl.: A61K 7/021, A61K 7/13, A61K 7/42, A61K 7/48, C08K 5/3417, C08L 5/08, C08L 33/12, C08L 35/04, C08L 83/04, C08L 89/04

(54) **Produits à base de particules de polymère comportant des pigments mélaniques, son procédé de préparation et son utilisation, en particulier en cosmétique**
Produkte auf der Basis von Melaninpigmente enthaltenden Polymerpartikeln, Verfahren zu ihrer Herstellung und ihre Verwendung, insbesondere in der Kosmetik
Products based on polymer particles containing melanine pigments, process for their preparation, and their use, particularly cosmetically

(30) Priorité: 17.01.1989 LU 87429
(43) Date de publication de la demande: 25.07.1990
(73) Titulaire: L'OREAL, 75008 Paris (FR)
(72) Inventeur: Grollier, Jean-François, F-75004 Paris (FR); Mahieu, Claude, F-75017 Paris (FR); Papantoniou, Christos, F-95160 Montmorency (FR)
(74) Mandataire: Casalonga, Axel

(56) Documents cités:
- EP-A- 0 313 380
- FR-A- 2 371 917
- GB-A- 2 197 885
- GB-A- 2 207 153

## Description

La présente invention est relative à des produits nouveaux constitués de particules fines de polymère, comportant des pigments mélaniques, à leur procédé de préparation et à leur utilisation, notamment dans le domaine de la cosmétique, pour le maquillage des poils et de la peau, la protection de l'épiderme humain contre le rayonnement UV et la coloration des cheveux.

La couleur des cheveux, de la peau et des poils d'origine humaine provient principalement des pigments mélaniques secrétés par les mélanocytes et l'on connaît bien, par ailleurs, le rôle protecteur du pigment mélanique vis-à-vis du rayonnement solaire.

Ces pigments, d'origine naturelle, comprennent en particulier des pigments noirs ou bruns que l'on appelle des eumélanines.

Leur biosynthèse naturelle s'effectue en plusieurs étapes par polymérisation des produits d'oxydation d'un acide aminé : la tyrosine et l'un de ses produits d'oxydation est le 5,6-dihydroxyindole qui polymérise à son tour en eumélanine.

Beaucoup de procédés de teinture des fibres kératiniques utilisant des dérivés indoliques ont été proposés dans la passé.

Le brevet GB 2 197 885 décrit un procédé de teinture des fibres kératiniques, mettant en oeuvre des précurseurs de colorants d'oxydation indoliques dont la polymérisation oxydative sur les fibres kératiniques à l'aide d'un système oxydant à base d'ions iodure et de péroxyde d'hydrogène, aboutit à un pigment mélanique synthétique procurant des teintures permanentes avec des couleurs intenses.

On a déjà cherché à fabriquer in vitro des pigments à base de pseudomélanine ou de composés similaires à la mélanine qui soient non toxiques et non allergènes et qui s'avèrent, notamment, intéressants dans les compositions de maquillage pour la peau, les poils, les cils et les sourcils, pour lesquels on recherche des pigments présentant une bonne innocuité par rapport aux pigments habituellement utilisés, tels que les pigments à base d'oxyde de fer.

La demanderesse a découvert, ce qui fait l'objet de l'invention, qu'il était possible de préparer avec un bon rendement, un produit constitué par des particules fines à base de polymères particuliers définis ci-après et d'un pigment mélanique.

On appelle pigment mélanique le pigment formé par oxydation du 5,6-dihydroxyindole, éventuellement associé au 2-carboxy 5,6-dihydroxyindole.

Par analogie et simplification, on appellera également "pigment mélanique" le pigment formé par oxydation de chacun des composés de formule (I) définie ci-après.

Ces produits nouveaux peuvent être utilisés en tant que pigments ou adjuvants dans divers domaines techniques où l'on a recours à l'utilisation de particules pigmentées, tels que le domaine de la peinture, de la cosmétique, ...

Les produits conformes à l'invention sont utilisés plus particulièrement comme pigments dans l'industrie cosmétique, notamment pour la protection de l'épiderme humain contre les effets néfastes du rayonnement solaire, pour le maquillage de la peau, des poils, des cils et des sourcils et la coloration des cheveux.

La présente invention a donc pour objet un produit constitué par des particules fines de polymères particuliers, comportant des pigments mélaniques.

Un autre objet de l'invention est constitué par la préparation d'un tel produit.

L'invention concerne également l'application cosmétique de tels produits, notamment dans le maquillage de la peau et des poils (cils et sourcils), la protection de l'épiderme humain contre le rayonnement UV et la coloration des cheveux.

D'autres objets de l'invention apparaîtront à la lecture de la description et des exemples qui suivent.

Le produit conforme à l'invention est essentiellement caractérisé par le fait qu'il est constitué de particules de polymères définis ci-après ayant une granulométrie inférieure à 100 µm et comportant, en surface et/ou dans le réseau polymère, un pigment mélanique naturel ou synthétique, formé in situ ou formé préalablement et absorbé par les particules polymères.

Le pigment mélanique résulte notamment de l'oxydation d'au moins un colorant indolique, répondant à la formule : 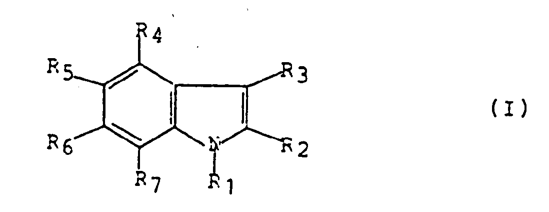
dans laquelle :
R₁ représente un atome d'hydrogène ou un groupement alkyle en C₁-C₄;
R₂ et R₃, identiques ou différents, représentent un atome d'hydrogène, un groupement alkyle en C₁-C₄, un groupement carboxyle ou un groupement alcoxy en C₁-C₄ carbonyle;
R₄, R₅, R₆ et R₇, identiques ou différents, représentent un atome d'hydrogène, un radical alkyle en C₁-C₄, un groupement -NHR (dans lequel R désigne H, alkyle en C₁-C₄, hydroxyalkyle en C₁-C₄, polyhydroxyalkyle en C₂-C₄), acylamino, carboxyle, carboxyalkyle en C₁-C₄, alcoxy C₁-C₄ carbonyle, alcoxy C₁-C₄ carbonylalkyle C₁-C₄, carbamyle, halogène, mono- ou polyhydroxyalkyle en C₁-C₄, aminoalkyle en C₁-C₄, un groupement OZ, dans lequel Z désigne hydrogène, alkyle linéaire ou ramifié en C₁-C₂₀, un groupement aralkyle (C₁-C₄), un groupement formyle, un groupement acyle en C₂-C₂₀ linéaire ou ramifié, un groupement alcénoyle en C₃-C₂₀ linéaire ou ramifié, un groupement -SiR₁₁R₁₂R₁₃, un groupement -P(O)(OR₈)₂, un groupement R₈OSO₂-; les radicaux R₄ et R₅, ou bien R₅ et R₆, ou bien R₆ et R₇ pouvant former, conjointement avec les atomes de carbone auxquels ils sont rattachés, un cycle contenant éventuellement un groupement carbonyle, un groupement thiocarbonyle, un groupement 〉P(O)(OR₈) ou un groupement 〉CR₉R₁₀;
sous réserve qu'au moins l'un des radicaux R₄ à R₇ représente un groupement OZ, ou bien que l'un des radicaux R₄ à R₇ représente -NHR, ou bien que R₄ et R₅, ou bien R₅ et R₆, ou bien R₆ et R₇ forment un cycle, R₈ et R₉ représentent un atome d'hydrogène ou un groupement alkyle inférieur en C₁-C₄, R₁₀ représente un groupement alcoxy C₁-C₄ ou un groupement mono- ou dialkyl(C₁-C₄) amino, R₁₁, R₁₂ et R₁₃, identiques ou différents, représentent des groupements alkyle C₁-C₄, linéaires ou ramifiés,
et les sels correspondants des métaux alcalins, alcalino-terreux, d'ammonium et d'amines.

Les colorants indoliques sont choisis de préférence parmi le 4-hydroxyindole, le 4-hydroxy 5-méthoxyindole, le 4-hydroxy 5-éthoxyindole, le 5-hydroxyindole, le 2-carboxy 5-hydroxyindole, le 5-hydroxy 6-méthoxyindole, le 6-hydroxyindole, le 6-hydroxy 7-méthoxyindole, le 5-méthoxy 6-hydroxyindole, le 7-hydroxyindole, le 5,6-dihydroxyindole, le 1-méthyl 5,6-dihydroxyindole, le 2-méthyl 5,6-dihydroxyindole, le 3-méthyl 5,6-dihydroxyindole, le 2,3-diméthyl 5,6-dihydroxyindole, le (5 ou 6)-acétoxy (6 ou 5)-hydroxyindole.

La granulométrie de ces particules est généralement supérieure à 0,01 µm et est de préférence comprise entre 0,01 et 50 µm et en particulier entre 0,1 et 20 µm.

Elles sont de préférence sphériques.

Les polymères utilisables selon l'invention sont des polymères insolubles dans le milieu réactionnel et sont choisis parmi les polymères naturels ou synthétiques, organiques ou inorganiques, à réseau réticulé, cristallin ou amorphe, ayant un poids moléculaire compris entre 5.000 et 5.000.000, définis ci-après.

Le caractère essentiellement insoluble du polymère est justifié par des raisons essentiellement économiques dans la mesure où le pigment mélanique doit se fixer sur un support particulaire solide, pour former le produit conforme à l'invention.

La solubilité des polymères dans le milieu réactionnel ne doit pas dépasser de préférence 10%.

Ces polymères naturels ou synthétiques sont choisis parmi:
a) les polymères dérivés de la kératine qui sont en particulier choisis parmi les kératines animales ou humaines, issues, par exemple, de matériaux choisis parmi les cheveux, la laine, la peau, les poils, les soies, les plumes, les écailles et plus particulièrement les sabots, la corne.
   Ces matériaux sont de préférence lavés et/ou dégraissés, puis réduits en particules.
   D'autres polymères dérivés de la kératine sont des kératines modifiées chimiquement, ayant un poids moléculaire compris entre 10.000 et 250.000, et en particulier la kératine partiellement hydrolysée (ou hydrolysat de kératine), obtenue à partir de peaux qui sont riches en produits soufrés et ayant un poids moléculaire compris entre 50.000 et 200.000. Cet hydrolysat est de préférence obtenu par hydrolyse alcaline modérée.
   Des produits de ce type sont par exemple vendus sous la dénomination de KERASOL par la Société CRODA.
   D'autres kératines modifiées sont les kératines sulfoniques d'un poids moléculaire compris entre 10.000 et 100.000, obtenues à partir de plumes d'oie ou de poulet ou plus avantageusement encore de sabots ou de corne.
   Cette kératine est obtenue par oxydation de tout ou partie des liaisons disulfures des groupements cystine de la kératine en groupements acide cystéique : SO₃H, l'oxydation étant avantageusement effectuée en milieu acide, tel que l'acide formique, au moyen d'un agent oxydant, tel que l'eau oxygénée.
b) La fibroïne de soie.
c) Les polymères dérivés de la chitine : ils sont constitués par la chitine qui est un polymère naturel, dont la source la plus importante se trouve dans la carapace des crustacés, tels que crabes, homards, langoustes, etc... La chitine est préparée selon un procédé décrit, notamment, dans l'ouvrage de R.A.A. MUZZARELLI "CHITIN", édité chez PERGAMON PRESS OXFORD, 1977, pages 89-100 et 207-217. On peut également utiliser son dérivé désacétylé connu sous la dénomination de chitosane, obtenu par saponification des groupements acétyle de la chitine.
   Le chitosane, tel que proposé dans le commerce, est particulièrement acétylé et contient 70 à 90% en poids de chitosane. On peut également l'utiliser sous forme de ses sels insolubles, tels que les sulfates et phosphates. Des produits de ce type sont vendus par exemple sous la dénomination de KYTEX par la Société HERCULES.
d) Les polymères synthétiques sont choisis parmi :
   (i) le polyméthracrylate de méthyle réticulé, tel que le produit vendu sous la dénomination MICROPEARL M 305 par la Société SEPPIC;
   (ii) la poly-β-alanine réticulée, telle que décrite dans le brevet français 2.530.250, ou encore présentée avantageusement sous forme de microsphères présentant une très faible dispersité de taille, 85% en poids ayant une granulométrie comprise entre 28 et 46 µm. Ces poly-β-alanines sont obtenues suivant un procédé consistant à polymériser entre 60 et 100°C, et de préférence vers 80°C, de l'acrylamide dans un mélange de solvants t-butanol/toluène, dans des rapports compris entre 1:24 et 10:1 et de préférence 1:6 et 6:1, en présence d'un initiateur de polymérisation, d'un copolymère octadécène-anhydride maléique comme agent de suspension, puis à soumettre la suspension de poly-β-alanine obtenue à une réticulation à l'aide d'un dialdéhyde, tel que la glutaraldéhyde.
      L'initiateur de polymérisation est de préférence du tertiobutylate de sodium ou de potassium (0,1 à environ 2 moles % par rapport à l'acrylamide).
      Le glutaraldéhyde est utilisé sous forme d'une solution aqueuse entre 20 et 25% et dans une proportion comprise entre 1 et 15% en poids et de préférence entre 1 et 8% en poids par rapport au poids d'acrylamide de départ;
   (iii) des microsphères creuses d'un copolymère de chlorure de vinylidène et d'acrylonitrile, vendues sous la dénomination EXPANCEL par la Société KEMA NORD;
   (iv) des microsphères poreuses de polyamide 12, de polyamide 6 ou de copolyamide 6/12, vendues sous la dénomination ORGASOL par la Société ATO-CHIMIE. Ces microsphères ont de préférence une granulométrie comprise entre 10 et 50 µm;
   (v) des poudres de silicone qui sont des gommes, des résines et plus particulièrement des élastomères d'organopolysiloxanes.

Les produits conformes à l'invention sont préparés suivant un procédé consistant essentiellement à mélanger à l'air et à une température de préférence ambiante et pouvant aller jusqu'à 100°C, le composé indolique de formule (I) et les particules des polymères décrits ci-dessus, dans un milieu essentiellement non-solvant du polymère, comme défini ci-dessus.

Si on n'utilise pas d'autres agents oxydants que l'oxygène de l'air, on opère à un pH de préférence alcalin, auquel cas le pigment se forme progressivement et se fixe sur la surface des particules et/ou dans le réseau ou les pores de celles-ci. On peut également utiliser en présence d'oxygène un catalyseur métallique d'oxydation tel que l'ion cuivrique.

Ces produits peuvent également être préparés en procédant à une formation immédiate du pigment mélanique, en utilisant un agent oxydant tel que le peroxyde d'hydrogène, l'acide periodique et ses sels hydrosolubles et dérivés, les permanganates et bichromates, tel que de sodium ou de potassium, l'hypochlorite de sodium, le ferricyanure de potassium, le persulfate d'ammonium, l'oxyde d'argent, le chlorure ferrique, l'oxyde de plomb (Pb IV), le nitrite de sodium, par addition d'iodure et de peroxyde d'hydrogène, l'iodure étant de préférence un iodure de métal alcalin, alcalino-terreux ou d'ammonium.

Ces agents oxydants peuvent être activés éventuellement par un agent modificateur de pH.

Pour les produits destinés à une application cosmétique, on utilise de préférence comme agents oxydants le peroxyde d'hydrogène, l'acide periodique et ses sels, le permanganate de potassium, l'hypochlorite de sodium, le persulfate d'ammonium, le nitrite de sodium et le système iodure/peroxyde d'hydrogène.

L'ordre d'addition des composés intervenant dans la préparation du produit, conforme à l'invention, a peu d'importance à la condition que l'on incorpore en dernier lieu l'agent oxydant quand celui-ci est utilisé sans agent modificateur de pH, et dans le cas du système oxydant iodure/peroxyde d'hydrogène, on introduit en dernier lieu, soit le peroxyde d'hydrogène, soit l'iodure.

Dans le cas où l'on utilise un agent modificateur de pH pour activer l'agent oxydant, on préfère ajouter en dernier lieu, soit l'agent oxydant, soit l'agent modificateur de pH.

Lorsqu'on utilise un hydrolysat de kératine, le pH du milieu doit de préférence être inférieur à 5, afin d'éviter la solubilisation de la kératine modifiée.

Lorsqu'on utilise une kératine sulfonique, le milieu est soit essentiellement alcoolique, soit aqueux, auquel cas le pH doit être inférieur à 7.

Lorsqu'on utilise le chitosane, le milieu aqueux doit avoir de préférence un pH supérieur à 5,8.

Comme indiqué ci-dessus, le milieu réactionnel est un milieu non solvant du polymère considéré. Il est de préférence constitué par de l'eau et il peut éventuellement être constitué par un mélange d'eau et de solvant, le solvant étant choisi parmi l'alcool éthylique, l'alcool isopropylique, l'alcool tertiobutylique, les éthers monométhylique, monoéthylique, monobutylique de l'éthylèneglycol, l'acétate du monoéthyléther de l'éthylèneglycol.

Ces solvant doivent, par ailleurs, pouvoir solubiliser le colorant indolique.

Lorsque le milieu est constitué par un mélange eau-solvant(s), les solvants sont présents dans des concentrations de préférences comprises entre 0,5 et 90% en poids par rapport au poids total de la composition et en particulier entre 2 et 50% en poids et de préférence entre 2 et 20% en poids.

Leur nature est choisie et leur proportion est ajustée en fonction des critères de solubilité des dérivés indoliques et du critère d'insolubilité du polymère.

Dans le procédé conforme à l'invention, on utilise de préférence le colorant indolique dans des proportions pondérales comprises entre 0,1 et 10% et de préférence entre 1 et 5% en poids, le polymère représentant 0,05 à 50% en poids et de préférence 4 à 30% en poids, le reste du mélange réactionnel étant généralement constitué par l'eau ou un mélange eau/solvant.

Les oxydants sont mis en oeuvre dans des quantités suffisantes pour oxyder le colorant indolique et former le pigment mélanique.

Lorsque l'on utilise l'ion iodure pour former le pigment mélanique, celui-ci est utilisé de préférence dans des proportions de 0,07 à 4% et en particulier entre 0,7 et 3%, en observant un rapport colorant indolique/I⁻ compris entre 0,6 et 6.

Les proportions sont déterminées par rapport au poids du milieu réactionnel.

On peut utiliser, pour la préparation des particules de polymère comportant un pigment mélanique, un pigment mélanique naturel ou synthétique préparé au préalable qui se fixe à la surface des particules ou est absorbé par celles-ci. Dans ce cas, le pigment mélanique finement divisé est dispersé dans un milieu non solvant du polymère. Après absorption du pigment, les particules sont séchées.

Le produit sous forme de particules est utilisé de préférence dans le domaine cosmétique dans lequel il se présente de préférence sous la forme sphérique. Il peut être additionné dans les supports cosmétiques classiques à une concentration comprise entre 0,2 et 35% en poids et de préférence entre 2 et 20% en poids par rapport au poids total de la composition, pour conduire à des compositions cosmétiques protectrices de l'épiderme humain, des produits de maquillage, notamment des cils, des sourcils ou de la peau, tels que des fards à paupières, fards à joues, liqueurs encore appelés "eye-liners", mascaras pour les cils et les sourcils, ou encore des compositions tinctoriales pour cheveux. Ces supports cosmétiques sont connus en eux-mêmes.

Ces compositions peuvent se présenter sous forme de lotion, de lotion épaissie, de gel, de crème, de lait, de poudre, de stick et éventuellement être conditionnées en aérosol et se présenter sous forme de mousse.

Lorsque les compositions sont utilisées pour le maquillage de la peau, des cils et des sourcils, elles peuvent notamment se présenter sous forme solide ou pâteuse, anhydre ou aqueuse, contre les émulsions huile-dans-eau ou eau-dans-huile ou encore les suspensions. Ces compositions présentent l'avantage d'être stables et de présenter une bonne innocuité.

Lorsque les compositions sont utilisées pour la protection de l'épiderme humain contre le rayonnement UV, elles constituent de ce fait des compositions dites "solaires", elles peuvent se présenter sous forme de suspensions ou de dispersions dans des solvants ou des corps gras, sous forme d'émulsions telles que crèmes et laits, de pommades, de gels, de bâtonnets solides ou de mousses aérosols.

Dans tous les cas, lorsqu'elles sont utilisées sous forme d'émulsions, elles peuvent contenir en outre des agents tensio-actifs bien connus dans l'état de la technique, choisis parmi les agents tensio-actifs anioniques, non ioniques, cationiques ou amphotères.

Les compositions de maquillage et les compositions solaires peuvent également contenir des corps gras, des solvants organiques, des silicones, des épaississants, des adoucissants, des filtres solaires, des agents anti-mousses, des agents hydratants, des parfums, des conservateurs, des agents anti-oxydants, des charges, des séquestrants, des agents de traitement tels que des polymères anioniques, cationiques, non ioniques, amphotères, ainsi que leurs mélanges, des propulseurs, des agents alcalinisants ou acidifiants.

Les corps gras peuvent être constitués par une huile ou une cire ou leur mélange, les acides gras, les alcools gras, la vaseline, la paraffine, la lanoline, la lanoline hydrogénée, la lanoline acétylée.

Les huiles sont choisies parmi les huiles animales, végétales, minérales ou de synthèse et notamment l'huile de palme hydrogénée, l'huile de ricin hydrogénée, l'huile de vaseline, l'huile de paraffine, l'huile de Purcellin.

Les cires sont choisies parmi les cires animales, fossiles, végétales, minérales ou synthétiques, parmi lesquelles on peut citer les cires d'abeille, les cires de Carnauba, de Candelilla, de canne à sucre, du Japon, les ozokérites, la cire de Montan, les cires microcristallines, les paraffines.

Les compositions peuvent également contenir en plus des particules ultrafines comportant des pigments mélaniques, telles que définies ci-dessus, d'autres pigments généralement utilisés en cosmétique, notamment les pigments nacrés permettant encore de varier les colorations susceptibles d'être obtenues.

Les exemples suivants sont destinés à illustrer l'invention sans pour autant présenter un caractère limitatif.

### EXEMPLE 1

On prépare une poudre de polymère et de pigment mélanique en additionnant, successivement sous agitation magnétique, dans un bécher, les ingrédients suivants :
- Poly-β-alanine réticulée à 3% (d'environ 2 µm) 4,9 g
- 5,6-dihydroxyindole 1,0 g
- Eau 91,4 g
- Nitrite de sodium 2,7 g
- HCl (1 N) qs pH = 4

Après filtration, lavage à l'eau puis à l'alcool éthylique, on obtient un poudre ultrafine noire.

### EXEMPLE 2

On prépare une poudre de polymère et de pigment mélanique en additionnant, successivement sous agitation magnétique, dans un bécher, les ingrédients suivants :
- 6-hydroxyindole 1,7 g
- Alcool éthylique 13,2 g
- Eau 69,5 g
- Iodure d'ammonium 1,1 g
- Poly-β-alanine réticulée (granulométrie comprise entre 0,1 et 1 µm) 9,9 g
- H₂O₂ (solution aqueuse à 20 volumes) 4,6 g

Après filtration, lavage à l'eau puis à l'alcool éthylique, on obtient une poudre ultrafine ocre rouge.

### EXEMPLE 3

On prépare un composé chitomélanique en additionnant, successivement sous agitation magnétique, dans un bécher, les ingrédients suivants :
- 5,6-dihydroxyindole 1,5 g
- Alcool éthylique 12,4 g
- Eau 73,1 g
- Iodure de potassium 0,7 g
- Chitine extraite de carapaces de crustacés, vendue sous la dénomination CHITIN LS 2970 par la Société Laboratoire Sérobiologique de Nancy 1,6 g
- Solution aqueuse de peroxyde d'hydrogène à 30 volumes 10,7 g

Le pigment se forme dès l'addition de l'oxydant.

Après filtration, lavage à l'eau puis à l'alcool éthylique, on obtient une poudre ultrafine noire.

### EXEMPLE 4

On prépare un composé chitomélanique en additionnant, successivement sous agitation magnétique dans un bécher, les ingrédients suivants :
- 5,6-dihydroxyindole 1,1 g
- Eau 96,0 g
- Chitine extraite de carapaces de crustacés, vendue par la Société Laboratoire Sérobiologique de Nancy sous la dénomination CHITIN LS 2970 1,9 g
- Periodate de sodium 1,0 g

Le pigment se forme dès l'addition de l'oxydant.

Après filtration, lavage à l'eau puis à l'alcool éthylique, on obtient une poudre ultrafine noire.

### EXEMPLE 5

On prépare un composé chitomélanique en additionnant, successivement sous agitation magnétique dans un bécher, les ingrédients suivants :
- 6-hydroxyindole 2,8 g
- Alcool éthylique 24,9 g
- Eau 69,2 g
- Chitine extraite de carapaces de crustacés, vendue par la Société Laboratoire Sérobiologique de Nancy sous la dénomination CHITIN LS 2970 1,4 g
- Persulfate d'ammonium 1,7g

Le pigment se forme dès l'addition de l'oxydant.

Après filtration, lavage à l'eau puis à l'alcool éthylique, on obtient une poudre ultrafine de teinte lie de vin.

### EXEMPLE 6

On prépare un composé chitomélanique en additionnant, successivement sous agitation magnétique dans un bécher, les ingrédients suivants :
- 5-méthoxy 6-hydroxyindole 0,8 g
- Alcool éthylique 31,7 g
- Eau 63,4 g
- Chitine extraite de carapaces de crustacés, vendue par la Société Laboratoire Sérobiologique de Nancy sous la dénomination CHITIN LS 2970 0,9 g
- Permangate de potassium en solution aqueuse 1 N : 3,2 g 0,51 g MA

Le pigment se forme dès l'addition de l'oxydant.

Après filtration, lavage à l'eau puis à l'alcool éthylique, on obtient une poudre ultrafine violet mauve cendré.

### EXEMPLE 7

On prépare un composé chitomélanique en additionnant, successivement sous agitation magnétique dans un bécher, les ingrédients suivants :
- 2,3-dimethyl 5,6-dihydroxyindole bromhydrate 3,0 g
- Alcool éthylique 27,6 g
- Eau 66,3 g
- Chitine extraite de carapaces de crustacés, vendue par la Société Sérobiologique de Nancy sous la dénomination CHITIN LS 2970 1,7 g
- Nitrite de sodium 1,4 g

La poudre ultrafine, après filtration, lavage à l'eau puis à l'alcool, est de teinte mauve.

### EXEMPLE 8

On prépare un composé chitomélanique en mélangeant, sous agitation magnétique, les composés suivants :
- Chitosane vendu sous la dénomination KYTEX M par la Société HERCULES 10,7 g
- Eau alcalinisée par NaOH
   Eau 75,9 g
   NaOH 1,4 g
- 5,6-dihydroxyindole 1,7 g
- Alcool éthylique 10,3 g

A la poudre de chitosane en suspension dans l'eau alcaline, on ajoute le 5,6-dihydroxyindole en solution dans l'alcool éthylique. On sépare le précipité noir obtenu par filtration, lavage à l'eau, puis à l'alcool éthylique.

### EXEMPLE 9

On prépare un composé kératino-mélanique en additionnant, successivement sous agitation magnétique, dans un bécher, les ingrédients suivants :
- Kératine (cheveux bruns lavés et réduits en poudre) 2,1 g
- 5,6-dihydroxyindole 0,8 g
- Alcool éthylique 21,1 g
- Eau 76,0 g

On laisse parfaire la réaction à l'air (15 minutes) avant de filtrer la poudre ultrafine gris beige obtenue.

### EXEMPLE 10

On prépare un composé kératino-mélanique en additionnant, successivement sous agitation magnétique, dans un bécher, les ingrédients suivants :
- 5,6-dihydroxyindole 1,0 g
- Alcool éthylique 24,4 g
- Eau 57,0 g
- Kératine (cheveux bruns lavés et réduits en poudre) 1,3 g
- NaOH en solution aqueuse 1 N (16,3 g) 0,65 g MA

On obtient après filtration, lavage à l'eau puis à l'alcool éthylique, une poudre ultrafine gris moyen.

### EXEMPLE 11

On prépare un composé kératino-mélanique en additionnant, successivement sous agitation magnétique, dans un bécher, les ingrédients suivants :
- Kératine (cheveux blancs lavés et réduits en poudre) 0,8 g
- 7-hydroxyindole 1,0 g
- Iodiure de potassium 1,0 g
- Alcool éthylique 14,0 g
- Eau 69,2 g
- H₂O₂ (solution aqueuse à 30 volumes) 14,0 g

On obtient après filtration, lavage à l'eau puis à l'alcool éthylique, une poudre ultrafine brun très foncé.

### EXEMPLE 12

On prépare un composé kératinomélanique en additionnant, successivement sous agitation magnétique, dans un bécher, les ingrédients suivants :
- Kératine (cheveux blancs lavés et réduits en poudre) 1,9 g
- 4-hydroxyindole 1,2 g
- Alcool éthylique 23,1 g
- Eau 70,1 g
- Periodate de sodium 3,7 g

Le pigment se forme dès l'addition de l'oxydant.

Après filtration, lavage à l'eau puis à l'alcool éthylique, on obtient une poudre ultrafine noire bleutée.

### EXEMPLE 13

On prépare un composé kératinomélanique en additionnant, successivement sous agitation magnétique dans un bécher, les ingrédients suivants :
- Kératine (cheveux bruns lavés et réduits en poudre) 1,4 g
- 5-méthoxy 6-hydroxyindole 2,8 g
- Alcool éthylique 41,7 g
- Eau 51,4 g
- Permanganate de potassium (solution aqueuse 1 N : 2,7 g) 0,43 g MA

Le pigment se forme dès l'addition de l'oxydant.

Après filtration, lavage à l'eau puis à l'alcool éthylique, on obtient une poudre ultrafine noire.

### EXEMPLE 14

On prépare un composé kératinomélanique en additionnant, successivement sous agitation magnétique dans un bécher, les ingrédients suivants :
- Kératine (cheveux bruns lavés et réduits en poudre) 1,1 g
- 6-hydroxyindole 1,1 g
- Alcool éthylique 22,0 g
- Eau 74,7 g
- Persulfate d'ammonium 1,1 g

Le pigment se forme dès l'addition de l'oxydant.

Après filtration, lavage à l'eau puis à l'alcool éthylique, on obtient une poudre ultrafine noire.

### EXEMPLE 15

On prépare un composé kératinomélanique en additionnant, successivement sous agitation magnétique dans un bécher, les ingrédients suivants :
- Kératine (cheveux blancs lavés et réduits en poudre) 1,0 g
- 3-méthyl, 5,6-dihydroxyindole 1,0 g
- Alcool éthylique 9,8 g
- Eau 87,6 g
- Nitrite de sodium 0,6 g
- HCl (1 N) qs pH = 4,9

Le pigment se forme dès l'addition de l'oxydant.

Après filtration, lavage à l'eau puis à l'alcool éthylique, on obtient une poudre ultrafine brune.

### EXEMPLE 16

On prépare un composé kératinomélanique en additionnant, successivement sous agitation magnétique dans un bécher, les ingrédients suivants :
- Kératine (cheveux blancs lavés et réduits en poudre) 3,2 g
- 4-hydroxyindole 3,2 g
- Alcool éthylique 32,0 g
- Eau 60,8 g
- Periodate de sodium 0,8 g

Le periodate se forme dès l'addition de l'oxydant.

Après filtration, lavage à l'eau puis à l'alcool éthylique, on obtient une poudre ultrafine gris moyen bleuté.

### EXEMPLE 17

On prépare un composé kératinomélanique en additionnant, successivement sous agitation magnétique dans un bécher, les ingrédients suivants :
- Kératine (cheveux blancs lavés et réduits en poudre) 1,0 g
- 5-hydroxyindole 1,7 g
- Alcool éthylique 9,8 g
- Eau 86,5 g
- Persulfate d'ammonium 1,0 g

Le pigment se forme dès l'addition de l'oxydant.

Après filtration, lavage à l'eau puis à l'alcool éthylique, on obtient une poudre ultrafine brun olive.

### EXEMPLE 18

On prépare un composé kératinomélanique en additionnant, successivement sous agitation magnétique dans un bécher, les ingrédients suivants :
- Kératine sulfonique (issue de sabots de bovins) 4,3 g
- 5,6-dihydroxyindole 0,9 g
- Iodure de sodium 0,9 g
- Alcool éthylique 87,0 g
- H₂O₂ (solution aqueuse à 30 volumes) 2,2 g

Le pigment se forme dès l'addition du peroxyde d'hydrogène.

Après filtration, lavage à l'eau puis à l'alcool éthylique, on obtient une poudre ultrafine noire.

### EXEMPLE 19

On prépare un composé kératinomélanique en additionnant, successivement sous agitation magnétique dans un bécher, les ingrédients suivants :
- Kératine sulfonique (issue de sabots de bovins) 6,1 g
- 6-hydroxyindole 1,8 g
- Iodure d'ammonium 0,4 g
- Alcool éthylique 81,9 g
- H₂O₂ (solution aqueuse à 30 volumes) 9,8 g

Le pigment se forme dès l'addition du peroxyde d'hydrogène.

Après filtration, lavage à l'alcool éthylique, on obtient une poudre ultrafine brun foncé.

### EXEMPLE 20

### (I) Préparation de la poly-β-alanine réticulée.

Dans un réacteur de 3 litres, muni d'un agitateur de type "ancre" d'un diamètre de 90 mm, d'une arrivée d'azote, d'une ampoule d'addition et d'une tête de colonne à distiller, on introduit : 934 g de toluène, 1666 g de tertiobutanol et 1,4 g de copolymère anhydride maléique/octadécène (vendu sous la dénomination PA-18) par la Société GULF. Après chauffage de ce mélange à 70°C, on y ajoute 100 g d'acrylamide. On porte alors la température à 100°C et on distille 120 ml du mélange azéotrope eau/toluène/tertiobutanol. Après la fin de la distillation, on refroidit le mélange réactionnel à 80°C, et on ajuste la vitesse d'agitation à 550 tours/minute. On ajoute alors en 10 min une solution de 2,24 g de tertiobutylate de potassium dans 40 g de tertiobutanol. L'ampoule d'addition est rincée par 120 ml de toluène. Après 7 heures d'agitation à 80°C, on laisse revenir à température ambiante. On ajoute ensuite au mélange, goutte à goutte, 7,5 ml d'acide chlorhydrique 12 N.

A la suspension des microsphères obtenues, on ajoute sous vive agitation (550 tours/minute), à 50°C, 100 ml d'eau en 15 minutes, puis 15 ml d'une solution de glutaraldéhyde à 25% en 20 minutes. Après avoir maintenu l'agitation pendant 4 heures à cette température, on laisse revenir à la température ambiante. Après décantation, les solvants surnageants sont éliminés et les microsphères sont lavées deux fois par 500 ml d'éthanol. L'essorage après chaque lavage est effectué par centrifugation (3500 tours/minute). Un lavage par 15 litres d'eau est ensuite effectué en continu, puis l'eau est éliminée jusqu'à un volume final de mélange de 600 ml.

La poly-β-alanine réticulée est ensuite séchée par lyophilisation et l'on obtient 92 g de poudre blanche dont le diamètre des microsphères est en moyenne 0,37±0,2 µm.

Le taux de réticulation, c'est-à-dire le rapport glutaraldéhyde/acrylamide, est d'environ 3%.

### (II) Préparation de particules comportant le pigment mélanique

Mode opératoire (IIa)
   0,55 g de 5,6-dihydroxyindole sont dissous dans 40 g d'eau, puis on ajuste à pH:9 par une solution de NaOH (1N). On y introduit 10 g des microsphères préparées suivant (I) imbibées par quelques gouttes d'éthanol. On chauffe à 40°C, puis agite au rotovapor pendant 40 minutes. On obtient une dispersion incolore. On y introduit 1,25 g de CuSO₄.5H₂O en solution dans 10 g d'eau. Dans la suspension, de coloration brune, on fait barboter de l'oxygène pendant 2 heures. Les microsphères noircies sont laissées sous agitation pendant une nuit, à la température ambiante, sous atmosphère d'oxygène, puis lavées jusqu'à ce que les eaux de lavage soient incolores et neutres; les particules sont séchées par lyophilisation.
Mode opératoire (IIb)
   a) 40 g de microsphères préparées suivant (I) sont gonflées par une solution composée de 10 g de CuSO₄.5H₂O et de 400 g d'eau. Le mélange est mis sous agitation pendant 1 heure à température ambiante, puis lyophilisé.
   b) 0,02 g de 5,6-dihydroxyindole sont dissous dans 80 g d'eau, puis on ajuste à pH:9 par une solution de NaOH (1N). Dans cette solution, on disperse 20 g de microsphères préparées plus haut (a). L'oxydation de la suspension noircie est complétée suivant le mode opératoire (IIa).

### EXEMPLE 21

On prépare une poudre de polymère et de pigment mélanique, en additionnant successivement, sous agitation magnétique, dans un bécher, les ingrédients suivants :
- 5,6-dihydroxyindole 5,0 g
- Alcool éthylique 6,6 g
- Polyméthacrylate de méthyle réticulé, vendu sous la dénomination MICROPEARL M 305 par la Société SEPPIC 3,7 g
- Persulfate d'ammonium 1,5 g
- Eau qsp 100,0 g

Après filtration, lavage à l'eau puis à l'alcool, on obtient une poudre ultrafine gris très foncé.

### EXEMPLE 22

On prépare une poudre de polymère et de pigment mélanique en additionnant, successivement sous agitation magnétique dans un bécher, les ingrédients suivants :
- 5,6-dihydroxyindole 11,1 g
- Alcool éthylique 16,7 g
- Eau 55,5 g
- Polyamide 12 vendu sous la dénomination ORGASOL 2002 D Naturel par la Société ATO-CHIMIE 11,1 g
- NaOH pur 5,6 g

On laisse parfaire la réaction à l'air (4 heures) avant de filtrer la poudre ultrafine noire obtenue qu'on sépare par filtration, lavage à l'eau, puis à l'alcool.

### EXEMPLE 23

On prépare une poudre de polymère et de pigment mélanique en additionnant, successivement sous agitation magnétique dans un bécher, les ingrédients suivants :
- 5,6-dihydroxyindole 11,1 g
- Alcool éthylique 16,7 g
- Eau 55,5 g
- Copolymère de chlorure de vinylidène/acrylonitrile sous forme de microsphère, vendu sous la dénomination EXPANCEL 551 WU par la Société KEMA NORD 11,1 g
- NaOH pur 5,6 g

On laisse parfaire la réaction à l'air (1 heure) avant de filtrer la poudre ultrafine gris très foncé obtenue qu'on sépare par filtration, lavage à l'eau, puis à l'alcool.

### EXEMPLE 24

On prépare une composition de coloration pour le traitement de cheveux blancs, en procédant au mélange des ingrédients suivants :
- Produit préparé selon l'exemple 20 (IIa ou IIb) 1,0 g
- Acide polyacrylique réticulé, vendu sous la dénomination CARBOPOL 940 par la Société GOODRICH 0,7 g
- Copolymère N-vinylpyrrolidone-acétate de vinyle, vendu par la Société GAF sous la dénomination PVP/VA-S-630 2,0 g
- Ethanol 17,0 g
- Emulsion à base de silicone cationique, vendue par DOW CORNING sous la dénomination DC 929 0,1 g
- Triéthanolamine qsp pH = 6,6
- Eau qsp 100,0 g

On applique 5 g de cette composition sur une méche de cheveux blancs. Après séchage, les cheveux sont colorés dans une nuance gris moyen.

### EXEMPLE 25

On prépare un mascara de composition suivante :
- Stérarate de triéthanolamine 15,0 g
- Cire de Candelilla 8,0 g
- Cire de Carnauba 10,0 g
- Hydroxy éthyl cellulose 1,0 g
- Gomme arabique 1,0 g
- Produit selon l'exemple 18 8,0 g
- Parahydroxy benzoate de méthyle 0,15 g
- Parahydroxy benzoate de propyle 0,15 g
- Eau permutée qsp 100,0 g

Dans cet exemple, on peut remplacer le produit de l'exemple 18 par celui de l'exemple 19.

### EXEMPLE 26

On prépare un eye liner de composition suivante :
- Propylèneglycol 8,0 g
- Polyéthylèneglycol 5,0 g
- Myristate d'isopropanolamine 3,0 g
- Gomme de xanthane 1,0 g
- Alcool polyvinylique 0,5 g
- Lanoline hydrogénée 4,0 g
- Parahydroxy benzoate de méthyle 0,1 g
- Parahydroxy benzoate de propyle 0,1 g
- Parahydroxy benzoate d'éthyle 0,1 g
- Mica titane 5,0 g
- Pigment noir (produit selon l'exemple 12) 7,0 g
- Eau permutée qsp 100,0 g

Dans cet exemple, le produit de l'exemple 12 peut être remplacé par exemple par ceux des exemples 1, 3, 4, 6, 8, 11, 13, 14, 15, 18, 19.

### EXEMPLE 27

On prépare un fond de teint de composition suivante :
- Acide stéarique 2,2 g
- Stéarate de glycérol 3,2 g
- Huile de vaseline 4,0 g
- Palmitate d'isopropyle 9,0 g
- Palmitate d'éthyl-2 hexyle 8,0 g
- Parahydroxy benzoate de propyle 0,1 g
- Triéthanolamine 1,1 g
- Glycérine 3,0 g
- Propylèneglycol 2,0 g
- Alumino silicate de magnésium 1,0 g
- Carboxy méthyl cellulose sodique 0,2 g
- Imidazolidinyl urée 0,3 g
- Oxyde de fer jaune 2,0 g
- Oxyde de fer 1,4 g
- Produit selon l'exemple 14 0,5 g
- Dioxyde de titane 8,0 g
- Talc 5,0 g
- Parfum 0,3 g
- Eau permutée qsp 100,0 g

Dans cet exemple, le produit de l'exemple 14 peut être remplacé par ceux des exemples 2, 5, 9, 10, 11, 13, 14, 15, 20 ou 21.

### EXEMPLE 28

On prépare une composition solaire suivante :
- Octyl-2 dodécanol-1 10,0 g
- Stéarate de magnésium 4,0 g
- Cire d'abeille 5,0 g
- Lanoline hydrogénée 1,0 g
- Lanoline 4,0 g
- Sesquioléate de sorbitan vendu sous la dénomination ARLACEL 83 par la Société ICI 4,5 g
- Mélange de monodistéarate de glycérol et de stéarate de potassium (9317) 1,0 g
- Huile de vaseline 27,0 g
- Produit préparé selon l'exemple 20 (IIa) ou 20 (IIb) 5,0 g
- Conservateurs qs
- Parfum qs
- Eau qsp 100,0 g

### EXEMPLE 29

Dans un bécher et sous agitation magnétique, on dissout 1,34 g de 6-aminoindole dans une solution constituée de 140 ml d'eau et 18 ml d'alcool.

Le pH est ajusté à 6,3 par de l'acide acétique dilué. On ajoute alors 9 ml d'eau oxygénée à 20 volumes puis, sous azote, 48,4 g de poly-β-alanine réticulée (préparée selon l'exemple 20).

On laisse gonfler la poly-β-alanine réticulée pendant 2 heures, puis on ajoute goutte à goutte une solution d'iodure de potassium (0,75 g d'iodure dissout dans 7,5 cc d'eau).

On maintient le brassage pendant 2 heures supplémentaires, puis on filtre et on lave à l'eau.

On obtient 57,2 g d'une poudre noire.

### EXEMPLE 30

On prépare la composition suivante :
- Poudre de fibroïne de soie 10,0 g
- 5,6-dihydroxyindole 1,0 g
- Alcool éthylique 20,0 g
- H₂O₂ (20 volumes) 20,0 g
- Triéthanolamine qsp pH = 8
- Eau qsp 100,0 g

Les ingrédients sont ajoutés les uns après les autres sous agitation. La coloration de la fibroïne de soie débute lorsque le pH est amené à 8 par la triéthanolamine. Le mélange est laissé sous agitation pendant 1 heure.

La solution est donc filtrée sur verre fritté n°4 (le filtrat est limpide). La partie récupérée sur verre filtré est rincée successivement à l'eau et à l'alcool. La poudre obtenue est séchée. Elle est couleur vieux-rose (rose-gris).

### EXEMPLE 31

On prépare la composition suivante :
- Polydiméthylsiloxane en poudre, vendu sous la dénomination SILICONE POWDER X2-1605 par la Société DOW CORNING 10,0 g
- 4-hydroxyindole 1,0 g
- Alcool éthylique 10,0 g
- H₂O₂ (30 volumes) 10,0 g
- Ammoniaque à 22°Bé 10,0 g
- Eau qsp 100,0 g

Le pH de la solution est de 10,6.

Les ingrédients sont ajoutés les uns après les autres, sauf le monohydroxyindole qui est préalablement dispersé dans l'alcool.

La polymérisation oxydative de l'indole commence dès l'addition de l'ammoniaque.

Le mélange est laissé sous agitation pendant 10 minutes, puis la solution est filtrée au verre fritté n°4.

La poudre du fritté est lavée successivement à l'eau et à l'alcool jusqu'à ce que le jus de lavage soit incolore. La poudre séchée obtenue est de couleur gris foncé légèrement bleuté.

## Revendications

1. Produit constitué de particules de polymères, caractérisé par le fait que les particules de polymères sont choisies parmi les particules de :
(a) polymères dérivés de la kératine éventuellement modifiée;
(b) fibroïne de soie;
(c) polymères dérivés de la chitine éventuellement désacétylée;
(d) polymères synthétiques choisis parmi :
(i) le polyméthracrylate de méthyle réticulé;
(ii) la poly-β-alanine réticulée;
(iii) des microsphères creuses du copolymère de chlorure de vinylidène et d'acrylonitrile;
(iv) des microsphères poreuses de polyamide 12, de polyamide 6 ou de copolyamide 6/12;
(v) de poudres de silicone constituées par des gommes, des résines, des élastomères d'organopolysiloxanes,
ces particules ayant une granulométrie inférieure à 100 µm et comportant en surface et/ou dans le réseau polymère, un pigment mélanique naturel ou synthétique, ce pigment étant soit formé préalablement et absorbé par les particules, soit formé in situ par oxydation d'un colorant indolique.

2. Produit selon la revendication 1 caractérisé par le fait que le pigment mélanique résultant de l'oxydation d'au moins un colorant indolique répond à la formule : 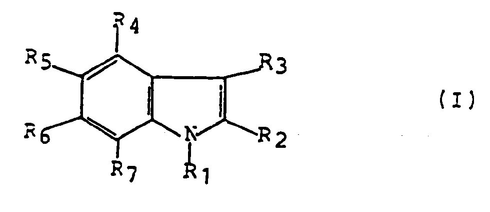 dans laquelle :
R₁ représente un atome d'hydrogène ou un groupement alkyle en C₁-C₄;
R₂ et R₃, identiques ou différents, représentent un atome d'hydrogène, un groupement alkyle en C₁-C₄, un groupement carboxyle ou un groupement alcoxy en C₁-C₄ carbonyle;
R₄, R₅, R₆ et R₇, identiques ou différents, représentent un atome d'hydrogène, un radical alkyle en C₁-C₄, un groupement -NHR (dans lequel R désigne H, alkyle en C₁-C₄, hydroxyalkyle en C₁-C₄, polyhydroxyalkyle en C₂-C₄), acylamino, carboxyle, carboxyalkyle en C₁-C₄, alcoxy C₁-C₄ carbonyle, alcoxy C₁-C₄ carbonylalkyle C₁-C₄, carbamyle, halogène, mono- ou polyhydroxyalkyle en C₁-C₄, aminoalkyle en C₁-C₄, un groupement OZ dans lequel Z désigne hydrogène, alkyle, linéaire ou ramifié en C₁-C₂₀, un groupement aralkyle (C₁-C₄), un groupement formyle, un groupement acyle en C₂-C₂₀, linéaire ou ramifié, un groupement alcénoyle en C₃-C₂₀, linéaire ou ramifié, un groupement alcénoyle en C₃-C₂₀, linéaire ou ramifié, un groupement -SiR₁₁R₁₂R₁₃, un groupement -P(O)(OR₈)₂, un groupement R₈OSO₂-; les radicaux R₄ et R₅, ou bien R₅ et R₆, ou bien R₆ et R₇ pouvant former, conjointement avec les atomes de carbone auxquels ils sont rattachés, un cycle contenant éventuellement un groupement carbonyle, un groupement thiocarbonyle, un groupement 〉P(O)(OR₈) ou un groupement 〉CR₉R₁₀; sous réserve qu'au moins l'un des radicaux R₄ à R₇ représente un groupement OZ, ou bien l'un des radicaux R₄ à R₇ représente -NHR, ou bien que R₄ et R₆, ou bien R₅ et R₆, ou bien R₆ et R₇ forment un cycle, R₈ et R₉ représentent un atome d'hydrogène ou un groupement alkyle inférieur en C₁-C₄, R₁₀ représente un groupement alcoxy en C₁-C₄ ou un groupement mono- ou dialkyl(C₁-C₄)amino, R₁₁, R₁₂ et R₁₃, identiques ou différents, représentent des groupements alkyle en C₁-C₄, linéaires ou ramifiés,
et les sels correspondants des métaux alcalins, alcalino-terreux, d'ammonium et d'amines.

3. Produit selon la revendication 1 ou 2, caractérisé par le fait que la granulométrie des particules est supérieure à 0,01 µm et elle est de préférence comprise entre 0,01 et 50 µm.

4. Produit selon la revendication 2, caractérisé par le fait que le colorant indolique est choisi parmi le 4-hydroxyindole, le 4-hydroxy 5-méthoxyindole, le 4-hydroxy 5-éthoxyindole, le 5-hydroxyindole, le 2-carboxy 5-hydroxyindole, le 5-hydroxy 6-méthoxyindole, le 6-hydroxyindole, le 6-hydroxy 7-méthoxyindole, le 5-méthoxy 6-hydroxyindole, le 7-hydroxyindole, le 5,6-dihydroxyindole, le 1-méthyl 5,6-dihydroxyindole, le 2-méthyl 5,6-dihydroxyindole, le 3-méthyl 5,6-dihydroxyindole, le 2,3-diméthyl 5,6-dihydroxyindole, le (5 ou 6)-acétoxy (6 ou 5)-hydroxyindole.

5. Produit selon l'une quelconque des revendications 1 à 4, caractérisé par le fait que lesdits polymères sont choisis parmi les polymères insolubles dans le milieu réactionnel, dans lequel se déroule la réaction d'oxydation et qu'ils sont à réseau réticulé, cristallin ou amorphe, et ont un poids moléculaire compris entre 5.000 et 5.000.000.

6. Produit selon l'une quelconque des revendications 1 à 5, caractérisé par le fait que les kératines sont choisies parmi les kératines animales ou humaines, susceptibles d'être issues de matériaux choisis parmi les cheveux, la laine, la peau, les poils, les soies, les plumes, les écailles, les sabots, la corne.

7. Produit selon l'une quelconque des revendications 1 à 5, caractérisé par le fait que le polymère dérivé de la kératine est une kératine modifiée chimiquement, ayant un poids moléculaire compris entre 10.000 et 250.000, par hydrolyse ou oxydation.

8. Produit selon la revendication 7, caractérisé par le fait que le polymère dérivé de la kératine est une kératine partiellement hydrolysée, susceptible d'être obtenue à partir de peaux et ayant un poids moléculaire compris entre 50.000 et 200.000.

9. Produit selon la revendication 7, caractérisé par le fait que la kératine modifiée est une kératine sulfonique, ayant un poids moléculaire compris entre 10.000 et 100.000, susceptible d'être obtenue à partir de plumes d'oie ou de poulet ou de sabots ou de corne.

10. Produit selon l'une quelconque des revendications 1 à 5, caractérisé par le fait que les polymères dérivés de la chitine sont constitués par la chitine ou son dérivé désacétylé appelé chitosane.

11. Produit selon l'une quelconque des revendications 1 à 5, caractérisé par le fait que le polymère est une poly-β-alanine réticulée.

12. Produit selon l'une quelconque des revendications 1 à 5, caractérisé par le fait que le polymère est constitué par des microsphères creuses de copolymères de chlorure de vinylidène et d'acrylonitrile ou de microsphères poreuses de polyamide 12, de polyamide 6 ou de copolyamide 6/12.

13. Procédé de préparation d'un produit tel que défini dans l'une quelconque des revendications à 12, caractérisé par le fait que soit on mélange en milieu aqueux le colorant indolique et une charge particulaire de polymère, ayant une granulométrie inférieure à 100 µm et qu'on procède ensuite à la formation du pigment par oxydation du colorant indolique, soit on disperse le pigment mélanique formé au préalable, sous forme finement divisée, dans un milieu non solvant du polymère et contenant lesdites particules de polymère, et après absorption du pigment, on sèche les particules.

14. Procédél selon la revendication 13, caractérisé par le fait que le colorant indolique mélangé à la charge particulaire de polymère est un colorant indolique tel que défini dans la revendication 2.

15. Procédé selon la revendication 13 ou 14, caractérisé par le fait que l'oxydation s'effectue lentement à l'air, à pH alcalin.

16. Procédé selon la revendication 13 ou 14, caractérisé par le fait que l'oxydation s'effectue par l'oxygène en présence d'un catalyseur métallique.

17. Procédé selon la revendication 13 ou 14, caractérisé par le fait que l'oxydation s'effectue par addition d'agents oxydants constitués par le peroxyde d'hydrogène, l'acide periodique et ses sels, les permanganates, les bichromates, l'hypochlorite de sodium, le ferricyanure de potassium, le persulfate d'ammonium, l'oxyde d'argent, le chlorure ferrique, l'oxyde de plomb (Pb IV), le nitrite de sodium.

18. Procédé selon la revendication 13 ou 14, caractérisé par le fait que l'oxydation s'effectue par addition d'un iodure alcalin, alcalino-terreux ou d'ammonium et de peroxyde d'hydrogène.

19. Procédé selon la revendication 13 ou 14 caractérisé par le fait que les agents oxydants sont choisis parmi le peroxyde d'hydrogène, l'acide périodique et ses sels, le permanganate de potassium, l'hypochlorite de sodium, le persulfate d'ammonium, le nitrite de sodium et le système iodure/peroxyde d'hydrogène.

20. Procédé selon l'une quelconque des revendications 13 à 19, caractérisé par le fait que le milieu réactionnel est un milieu non solvant du polymère considéré, constitué par de l'eau ou un mélange d'eau et de solvant (s).

21. Procédé selon la revendication 20 , caractérisé par le fait que le solvant est choisi parmi l'alcool éthylique, l'alcool isopropylique, l'alcool tertiobutylique, les éthers monométhylique, monoéthylique, monobutylique de l'éthylèneglycol, l'acétate du monéthyléther de l'éthylèneglycol.

22. Procédé selon l'une quelconque des revendications 13 à 21, caractérisé par le fait que le colorant indolique est présent dans des proportions pondérales comprises entre 0,1 et 10 % en poids et de préférence entre 1 et 5% en poids par rapport au poids du milieu réactionnel, le polymère représentant 0,05 à 50% en poids et de préférence 4 à 30% en poids par rapport au poids du mélange réactionnel.

23. Composition cosmétique, caractérisée par le fait qu'elle contient dans un milieu cosmétiquement acceptable, au moins un produit tel que défini dans l'une quelconque des revendications 1 à 12 ou susceptible d'être préparé selon le procédé défini dans l'une quelconque des revendications 13 à 22.

24. Composition selon la revendication 23, caractérisée par le fait qu'elle se présente sous forme de lotion, de lotion épaissie, de gel, de crème, de lait, de poudre, de stick et qu'elle est éventuellement conditionnée en aérosol.

25. Composition selon la revendication 23, destinée à être utilisée pour le maquillage de la peau, des cils et des sourcils, caractérisée par le fait qu'elle se présente sous forme solide ou pâteuse, anhydre ou aqueuse.

26. Composition selon l'une quelconque des revendications 23 ou 25, destinée à la protection de l'épiderme humain contre les rayonnements solaires, caractérisée par le fait qu'elle se présente sous forme de suspension ou de dispersion dans des solvants ou des corps gras ou sous forme d'émulsion, de pommade, de gel, de bâtonnet solide ou de mousse aérosol.

27. Composition selon la revendication 23, caractérisée par le fait qu'elle contient des corps gras, des solvants organiques, des silicones, des épaississants, des adoucissants, des filtres solaires, des agents anti-mousses, des agents hydratants, des parfums, des conservateurs, des agents antioxydants, des charges, des séquestrants, des agents de traitement, des propulseurs, des agents alcalinisants ou acidifiants ou des corps gras.

28. Composition selon l'une quelconque des revendications 23 à 27, caractérisée par le fait qu'elle contient d'autres pigments permettant de varier les colorations.

29. Application cosmétique de la composition selon la revendication 23 ou 26, à la protection de l'épiderme humain.

30. Application de la composition selon la revendication 23, comme produit de maquillage.

31. Application de la composition selon la revendication 23, pour la coloration des cheveux humains.

## Claims

1. Product consisting of particles of polymers, characterised in that the particles of polymers are selected from particles of:
(a) polymers derived from keratin, optionally modified;
(b) silk fibroin;
(c) polymers derived from chitin, optionally deacetylated;
(d) synthetic polymers selected from:
(i) crosslinked poly(methyl methacrylate);
(ii) crosslinked poly-β-alanine;
(iii) hollow microspheres of the copolymer of vinylidene chloride and acrylonitrile;
(iv) porous microspheres of polyamide 12, polyamide 6 or copolyamide 6/12;
(v) silicone powders consisting of gums, resins, organopolysiloxane elastomers,
these particles having a particle size of less than 100 µm and containing, at the surface and/or in the polymer network, a natural or synthetic melanin pigment, this pigment being either formed beforehand and absorbed by the particles, or formed in situ by oxidation of an indole dye.

2. Product according to Claim 1, characterised in that the melanin pigment resulting from the oxidation of at least one indole dye corresponds to the formula: 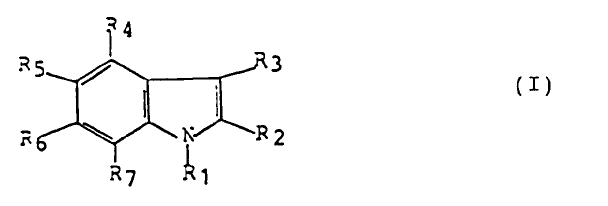 in which:
R₁ represents a hydrogen atom or a C₁-C₄ alkyl group;
R₂ and R₃, which may be identical or different, represent a hydrogen atom, a C₁-C₄ alkyl group, a carboxyl group or a (C₁-C₄ alkoxy)carbonyl group;
R₄, R₅, R₆ and R₇, which may be identical or different, represent a hydrogen atom, a C₁-C₄ alkyl radical, a group -NHR (in which R denotes H, C₁-C₄ alkyl, C₁-C₄ hydroxyalkyl, C₂-C₄ polyhydroxyalkyl), an acylamino, carboxyl, C₁-C₄ carboxyalkyl, (C₁-C₄ alkoxy)carbonyl, (C₁-C₄ alkoxy)carbonyl(C₁-C₄ alkyl), carbamyl, halogen, C₁-C₄ mono- or polyhydroxyalkyl or C₁-C₄ aminoalkyl group, a group OZ, in which Z denotes hydrogen, C₁-C₂₀ linear or branched alkyl, an aryl(C₁-C₄ alkyl) group, a formyl group, a linear or branched C₂-C₂₀ acyl group, a linear or branched C₃-C₂₀ alkenoyl group, a linear or branched C₃-C₂₀ alkenoyl group, (sic) a group -SiR₁₁R₁₂R₁₃, a group -P(O)(OR₈)₂, or a group R₈OSO₂-; it being possible for the radicals R₄ and R₅, or alternatively R₅ and R₆, or alternatively R₆ and R₇, to form, together with the carbon atoms to which they are attached, a ring optionally containing a carbonyl group, a thiocarbonyl group, a group 〉 P(O)(OR₈) or a group 〉CR₉R₁₀; with the proviso that at least one of the radicals R₄ to R₇ represents a group OZ, or alternatively that one of the radicals R₄ to R₇ represents -NHR, or alternatively that R₄ and R₆ (sic) or alternatively R₅ and R₆ or alternatively R₆ and R₇ form a ring, R₈ and R₉ represent a hydrogen atom or a C₁-C₄ lower alkyl group, R₁₀ represents a C₁-C₄ alkoxy group or a (C₁-C₄ mono- or dialkyl)amino group, and R₁₁, R₁₂ and R₁₃, which may be identical or different, represent linear or branched C₁-C₄ alkyl groups,
and the corresponding alkali metal, alkaline earth metal, ammonium and amine salts.

3. Product according to Claim 1 or 2, characterised in that the size of the particles is greater than 0.01 µm and is preferably between 0.01 and 50 µm.

4. Product according to Claim 2, characterised in that the indole dye is selected from 4-hydroxyindole, 4-hydroxy-5-methoxyindole, 4-hydroxy-5-ethoxyindole, 5-hydroxyindole, 2-carboxy-5-hydroxyindole, 5-hydroxy-6-methoxyindole, 6-hydroxyindole, 6-hydroxy-7-methoxyindole, 5-methoxy-6-hydroxyindole, 7-hydroxyindole, 5,6-dihydroxyindole, 1-methyl-5,6-dihydroxyindole, 2-methyl-5,6-dihydroxyindole, 3-methyl-5,6-dihydroxyindole, 2,3-dimethyl-5,6-dihydroxyindole and (5 or 6)-acetoxy-(6 or 5)-hydroxyindole.

5. Product according to any one of Claims 1 to 4, characterised in that the said polymers are selected from polymers which are insoluble in the reaction medium in which the oxidation reaction proceeds, and in that they have a crosslinked, crystalline or amorphous network and a molecular weight of between 5,000 and 5,000,000.

6. Product according to any one of Claims 1 to 5, characterised in that the keratins are selected from animal or human keratins capable of originating from materials selected from the hair, wool, skin, hairs, bristles, feathers, scales, hooves and horn.

7. Product according to any one of Claims 1 to 5, characterised in that the polymer derived from keratin is a keratin modified chemically by hydrolysis or oxidation, having a molecular weight of between 10,000 and 250,000.

8. Product according to Claim 7, characterised in that the polymer derived from keratin is a partially hydrolysed keratin capable of being obtained from skins and having a molecular weight of between 50,000 and 200,000.

9. Product according to Claim 7, characterised in that the modified keratin is a sulphonic keratin having a molecular weight of between 10,000 and 100,000, capable of being obtained from goose or chicken feathers or from hooves or horn.

10. Product according to any one of Claims 1 to 5, characterised in that the polymers derived from chitin consist of chitin or its deacetylated derivative known as chitosan.

11. Product according to any one of Claims 1 to 5, characterised in that the polymer is a crosslinked poly-β-alanine.

12. Product according to any one of Claims 1 to 5, characterised in that the polymer consists of hollow microspheres of copolymers of vinylidene chloride and acrylonitrile, or porous microspheres of polyamide 12, polyamide 6 or copolyamide 6/12.

13. Process for preparing a product as defined in any one of Claims [lacuna] to 12, characterised in that either the indole dye and a particulate charge of polymer having a particle size of less than 100 µm are mixed in an aqueous medium, and in that (sic) formation of the pigment is then carried out by oxidation of the indole dye, or the melanin pigment formed beforehand is dispersed, in finely divided form, in a medium which is non-solvent for the polymer and containing the said particles of polymer, and, after absorption of the pigment, the particles are dried.

14. Process according to Claim 13, characterised in that the indole dye mixed with the particulate charge of polymer is an indole dye as defined in Claim 2.

15. Process according to Claim 13 or 14, characterised in that the oxidation is performed slowly in the air at alkaline pH.

16. Process according to Claim 13 or 14, characterised in that the oxidation is performed with oxygen in the presence of a metal catalyst.

17. Process according to Claim 13 or 14, characterised in that the oxidation is performed by the addition of oxidising agents consisting of hydrogen peroxide, periodic acid and its salts, permanganates, dichromates, sodium hypochlorite, potassium ferricyanide, ammonium persulphate, silver oxide, ferric chloride, lead (PbIV) oxide or sodium nitrite.

18. Process according to Claim 13 or 14, characterised in that the oxidation is performed by the addition of an alkali metal iodide, alkaline earth metal iodide or ammonium iodide and hydrogen peroxide.

19. Process according to Claim 13 or 14, characterised in that the oxidising agents are selected from hydrogen peroxide, periodic acid and its salts, potassium permanganate, sodium hypochlorite, ammonium persulphate, sodium nitrite and the iodide/hydrogen peroxide system.

20. Process according to any one of Claims 13 to 19, characterised in that the reaction medium is a medium which is non-solvent for the polymer in question, consisting of water or a mixture of water and solvent(s).

21. Process according to Claim 20, characterised in that the solvent is selected from ethyl alcohol, isopropyl alcohol, tert-butyl alcohol, ethylene glycol monomethyl, monoethyl and monobutyl ethers and ethylene glycol monoethyl ether acetate.

22. Process according to any one of Claims 13 to 21, characterised in that the indole dye is present in proportions by weight of between 0.1 and 10% by weight, and preferably between 1 and 5% by weight, relative to the weight of the reaction medium, the polymer representing 0.05 to 50% by weight, and preferably 4 to 30% by weight, relative to the weight of the reaction mixture.

23. Cosmetic composition, characterised in that it contains, in a cosmetically acceptable medium, at least one product as defined in any one of Claims 1 to 12 or which is capable of being prepared according to the process defined in any one of Claims 13 to 22.

24. Composition according to Claim 23, characterised in that it is presented in the form of a lotion, thickened lotion, gel, cream, milk, powder or stick, and that it is optionally packaged as an aerosol.

25. Composition according to Claim 23, intended for use for makeup of the skin, eyelashes and eyebrows, characterised in that it is presented in anhydrous or aqueous, solid or pasty form.

26. Composition according to either of Claims 23 or 25, intended for protection of the human epidermis against solar radiation, characterised in that it is presented in the form of a suspension or dispersion in solvents or fats or in the form of an emulsion, ointment, gel, solid stick or aerosol foam.

27. Composition according to Claim 23, characterised in that it contains fats, organic solvents, silicones, thickeners, emollients, sunscreens, antifoams, moisturisers, fragrances, preservatives, antioxidants, fillers, sequestering agents, treatment agents, propellants, alkalinising or acidifying agents or fats (sic).

28. Composition according to any one of Claims 23 to 27, characterised in that it contains other pigments permitting variation of the colorations.

29. Cosmetic application of the composition according to Claim 23 or 26, for protection of the human epidermis.

30. Application of the composition according to Claim 23, as a makeup product.

31. Application of the composition according to Claim 23, for the dyeing of human hair.

## Patentansprüche

1. Produkt aus Polymerteilchen, dadurch gekennzeichnet, daß die Polymerteilchen ausgewählt sind aus den Teilchen von:
(a) Polymerderivaten von gegebenenfalls modifiziertem Keratin;
(b) Seidenfibroin;
(c) Polymerderivaten von gegebenenfalls deacetyliertem Chitin;
(d) synthetischen Polymeren, ausgewählt aus:
(i) vernetztem Polymethylmethacrylat;
(ii) vernetztem Poly-β-Alanin;
(ii) hohlen Mikrokugeln von Vinylidenchlorid/Acrylnitril-Copolymer;
(iv) porösen Mikrokugeln aus Polyamid 12, Polyamid 6 oder Copolyamid 6/12;
(v) Silikonpulvern aus Gummi, Harzen, Elastomeren von Organopolysiloxanen,
wobei die Teilchen eine Korngrößenverteilung von unterhalb 100 µm besitzen und an der Oberfläche und/oder im Polymerinneren ein natürliches oder synthetisches Pigment aufweisen, wobei dieses Pigment entweder vorher gebildet und durch die Teilchen absorbiert wurde, oder in situ durch Oxidation eines Indolfarbstoffes gebildet wurde.

2. Produkt nach Anspruch 1, dadurch gekennzeichnet, daß das Melaninpigment durch Oxidation mindestens eines Indolfarbstoffes der Formel erhalten wird: 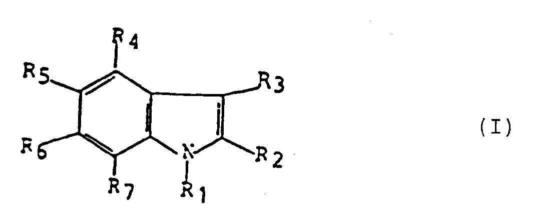 worin bedeuten:
R₁ ein Wasserstoffatom oder eine C₁-C₄-Alkylgruppe; R₂ und R₃ gleich oder verschieden sind, und ein Wasserstoffatom, eine C₁-C₄-Alkylgruppe, eine Carboxylgruppe oder eine C₁-C₄-Alkoxycarbonylgruppe bedeuten;
R₄, R₅, R₆ und R₇ gleich oder verschieden sind und ein Wasserstoffatom, ein C₁-C₄-Alkylradikal, eine Gruppe -NHR (worin R H, C₁-C₄-Alkyl, C₁-C₄-Hydroxyalkyl, C₂-C₄-Polyhydroxyalkyl bedeuten), Acylamino, Carboxyl, C₁-C₄-Carboxyalkyl, C₁-C₄-Alkoxycarbonyl, C₁-C₄-Alkoxy-carbonyl-C₁-C₄-alkyl, Carbamoyl, Halogen, C₁-C₄-Mono- oder Polyhydroxyalkyl, C₁-C₄-Aminoalkyl, eine Gruppe OZ, worin Z Wasserstoff, geradkettiges oder verzweigtes C₁-C₂₀-Alkyl, eine Ar-(C₁-C₄)-alkylgruppe, eine Formylgruppe, eine lineare oder verzweigte C₂-C₂₀-Acylgruppe, eine lineare oder verzweigte C₃-C₂₀-Alkenylgruppe, eine Gruppe -SiR₁₁R₁₂R₁₃, eine Gruppe - P(O)(OR₈)₂, eine Gruppe R₈OSO₂-; wobei die Reste R₄ und R₅, oder R₅ und R₆, oder R₆ und R₇ zusammen mit den Kohlenstoffatomen, an die sie gebunden sind, einen gegebenenfalls eine Carbonylgruppe, eine Thiocarbonylgruppe, eine Gruppe 〉P(O)(OR₈) oder eine Gruppe 〉CR₉R₁₀ enthaltenden Ring bilden können; mit der Maßgabe, daß mindestens einer der Reste R₄ bis R₇ eine Gruppe OZ darstellt, oder einer der Reste R₄ bis R₇ -NHR bedeutet, oder R₄ und R₆, R₅ und R₆ oder R₆ und R₇ einen Ring bilden, R₈ und R₉ ein Wasseratom oder eine niedere C₁-C₄-Alkylgruppe bedeuten, R₁₀ eine C₁-C₄-Alkoxygruppe oder eine Mono- oder Di-(C₁-C₄)-alkylaminogruppe bedeutet, R₁₁, R₁₂ und R₁₃, die gleich oder verschieden sind, eine geradkettige oder verzweigte C₁-C₄-Alkylgruppe bedeuten,
und den entsprechenden Alkalimetall-, Erdalkalimetall-, Ammonium- und Aminsalzen.

3. Produkt nach Anspruch 1 oder 2, dadurch gekennzeichnet, daß die Korngrößenverteilung der Teilchen oberhalb 0,01 µm liegt, und insbesondere zwischen 0,01 und 50 µm.

4. Produkt nach Anspruch 2, dadurch gekennzeichnet, daß der Indolfarbstoff ausgewählt ist aus 4-Hydroxyindol, 4-Hydroxy-5-methoxyindol, 4-Hydroxy-5-ethoxyindol, 5-Hydroxyindol, 2-Carboxy-5-hydroxyindol, 5-Hydroxyindol, 5-Hydroxy-6-methoxyindol, 6-Hydroxyindol, 6-Hydroxy-7-methoxyindol, 5-Methoxy-6-hydroxyindol, 7-Hydroxyindol, 5,6-Dihydroxyindol, 1-Methyl-5,6-dihydroxyindol, 2-Methyl-5,6-dihydroxyindol, 3-Methyl-5,6-dihydroxyindol, 2,3-Dimethyl-5,6-dihydroxyindol, (5 oder 6)-Acetoxy-(6 oder 5)-hydroxyindol.

5. Produkt nach einem der Ansprüche 1 bis 4, dadurch gekennzeichnet, daß die Polymeren ausgewählt sind unter den Polymeren, die im Reaktionsmilieu, in dem sie der Oxidationsreaktion unterliegen, unlöslich sind, und die eine kristalline oder amorphe vernetzte Struktur aufweisen, und ein Molekulargewicht zwischen 5000 und 5000000 besitzen.

6. Produkt nach einem der Ansprüche 1 bis 5, dadurch gekennzeichnet, daß die Keratine ausgewählt sind unter den tierischen oder menschlichen Keratinen, und aus Materialien ausgewählt unter Haaren, Wolle, Haut, Körperhaaren, Seiden, Federn, Schuppen, Hufen, Horn stammen können.

7. Produkt nach einem der Ansprüche 1 bis 5, dadurch gekennzeichnet, daß das polymere Keratinderivat ein durch Hydrolyse oder Oxidation chemisch modifiziertes Keratin mit einem Molekulargewicht zwischen 10000 und 250000 ist.

8. Produkt nach Anspruch 7, dadurch gekennzeichnet, daß das polymere Keratinderivat ein partiell hydrolysiertes Keratin ist, das aus Haut erhalten werden kann, und ein Molekulargewicht zwischen 50000 und 200000 besitzt.

9. Produkt nach Anspruch 7, dadurch gekennzeichnet, daß das modifizierte Keratin ein sulfoniertes Keratin ist, das aus Gänse- oder Hühnerfedern oder Hufen oder Horn erhalten werden kann, mit einem Molekulargewicht zwischen 10000 und 100000.

10. Produkt nach einem der Ansprüche 1 bis 5, dadurch gekennzeichnet, daß das polymere Chitinderivat aus Chitin oder seinem als Chitosan bezeichneten deacetylierten Derivat aufgebaut ist.

11. Produkt nach einem der Ansprüche 1 bis 5, dadurch gekennzeichnet, daß das Polymere ein vernetztes Poly-β-Alanin ist.

12. Produkt nach einem der Ansprüche 1 bis 5, dadurch gekennzeichnet, daß das Polymere aus hohlen Mikrokugeln von Vinylidenchlorid/Acrylnitril-Copolymeren oder aus porösen Mikrokugeln von Polyamid 12, Polyamid 6 oder Copolyamid 6/12 aufgebaut ist.

13. Verfahren zur Herstellung eines in einem der Ansprüche 1 bis 12 definierten Produktes, dadurch gekennzeichnet, daß man entweder in einem wäßrigen Milieu den Indolfarbstoff und ein teilchenförmiges Polymer mit einer Korngrößenverteilung unterhalb von 100 µm mischt und dann die Bildung des Pigmentes durch Oxidation des Indolfarbstoffes durchführt, oder das vorher gebildete Melaninpigment in feinverteilter Form in einem Milieu, das für das Polymer kein Lösungsmittel ist und die Polymerteilchen enthält, dispergiert und die Teilchen nach Absorption des Pigmentes trocknet.

14. Verfahren nach Anspruch 13, dadurch gekennzeichnet, daß der mit einem teilchenförmigen Polymer gemischte Indolfarbstoff ein Indolfarbstoff, wie er in Anspruch 2 definiert ist, ist.

15. Verfahren nach Anspruch 13 oder 14, dadurch gekennzeichnet, daß die Oxidation langsam an der Luft bei einem alkalischen pH-Wert durchgeführt wird.

16. Verfahren nach Anspruch 13 oder 14, dadurch gekennzeichnet, daß die Oxidation durch Sauerstoff in Gegenwart eines Metallkatalysators durchgeführt wird.

17. Verfahren nach Anspruch 13 oder 14, dadurch gekennzeichnet, daß die Oxidation durch Zugabe eines Oxidationsmittels durchgeführt wird, das aus Wasserstoffperoxid, Perjodsäure und ihren Salzen, den Permanganaten, den Bichromaten, Natriumhypochlorit, Kaliumferricyanid, Ammoniumpersulfat, Silberoxid, Ferrichlorid, Bleioxid (Pb IV), Natriumnitrit besteht.

18. Verfahren nach Anspruch 13 oder 14, dadurch gekennzeichnet, daß die Oxidation durch Zugabe eines Alkali-, Erdalkali- oder Ammoniumjodids und Wasserstoffperoxid durchgeführt wird.

19. Verfahren nach Anspruch 13 oder 14, dadurch gekennzeichnet, daß die Oxidationsmittel ausgewählt sind unter Wasserstoffperoxid, Perjodsäure und ihren Salzen, Kaliumpermanganat, Natriumhypochlorit, Ammoniumpersulfat, Natriumnitrit und dem System Jodid/Wasserstoffperoxid.

20. Verfahren nach einem der Ansprüche 13 bis 19, dadurch gekennzeichnet, daß das Reaktionsmilieu ein Milieu ist, das für das in Betracht gezogene Polymere kein Lösungsmittel darstellt, und aus Wasser oder einer Mischung aus Wasser und Lösungsmittel(n) besteht.

21. Verfahren nach Anspruch 20, dadurch gekennzeichnet, daß das Lösungsmittel ausgewählt ist aus Ethylalkohol, Isopropylalkohol, tert.-Butylalkohol, den Monomethyl-, Monoethyl- oder Monobutylethern von Ethylenglykol, dem Ethylenglykolmonoethyletheracetat.

22. Vefahren nach einem der Ansprüche 13 bis 21, dadurch gekennzeichnet, daß der Indolfarbstoff in Gewichtsanteilen zwischen 0,1 und 10 Gew.-%, und vorzugsweise zwischen 1 und 5 Gew.-%, bezogen auf das Gewicht des Reaktionsmilieus, vorhanden ist, und der Anteil des Polymeren 0,05 bis 50 Gew.-%, und vorzugsweise 4 bis 30 Gew.-%, bezogen auf das Gewicht der Reaktionsmischung, beträgt.

23. Kosmetische Zusammensetzung, dadurch gekennzeichnet, daß sie in einem kosmetisch annehmbaren Milieu mindestens ein Produkt enthält, wie es in einem der Ansprüche 1 bis 12 definiert ist oder das nach einem in einem der Ansprüche 13 bis 22 definierten Verfahren hergestellt werden kann.

24. Zusammensetzung nach Anspruch 23, dadurch gekennzeichnet, daß sie in Form einer Lotion, einer verdickten Lotion, eines Gels, einer Creme, einer Milch, eines Puders, eines Stiftes vorliegt oder gegebenenfalls in einem Aerosol konditioniert ist.

25. Zusammensetzung nach Anspruch 23, bestimmt zur Verwendung zum Schminken der Haut, der Wimpern und der Augenbrauen, dadurch gekennzeichnet, daß es in wasserfreier oder wäßriger fester oder pastenförmiger Form vorliegt.

26. Zusammensetzung nach einem der Ansprüche 23 bis 25 zum Schutz der menschlichen Epidermis gegen Sonnenstrahlen, dadurch gekennzeichnet, daß es in Form einer Suspension oder Dispersion in einem Lösungsmittel oder in einem Fettkörper oder in Form einer Emulsion, einer Salbe, eines Gels, eines festen Stiftes oder als Aerosolschaum vorliegt.

27. Zusammensetzung nach Anspruch 23, dadurch gekennzeichnet, daß es Fettkörper, organische Lösungsmittel, Silikone, Verdickungsmittel, Weichmacher, Sonnenfilter, Antischaummittel, Hydratationsmittel, Parfüms, Konservierungsstoffe, Antioxidantien, Füllstoffe, Sequistriermittel, Behandlungsmittel, Treibmittel, alkalisch oder sauermachende Mittel oder Fettkörper enthält.

28. Zusammensetzung nach einem der Ansprüche 23 bis 27, dadurch gekennzeichnet, daß es andere Pigmente enthält, die eine Variation der Färbungen erlauben.

29. Kosmetische Verwendung der Zusammensetzung nach Anspruch 23 oder 26 zum Schutz der menschlichen Epidermis.

30. Vewendung der Zusammensetzung nach Anspruch 23 als Schminkprodukt.

31. Verwendung der Zusammensetzung nach Anspruch 23 zum Färben menschlicher Haare.
